# EUROPEAN PATENT APPLICATION

(11) **EP 3 714 959 A1**
(43) Date of publication of application: **30.09.2020**
(21) Application number: 19166238.6
(22) Date of filing: 29.03.2019
(51) Int. Cl.: B01D 15/08, B01D 15/36, C12P 15/00, C12P 17/18, C12R 1/645, A01N 43/56, A01N 37/42

(54) **ISOLATION OF SMALL MOLECULES FROM THE FERMENTATION BROTH OF A EUKARYOTIC MICROORGANISM**

(71) Applicant: BASF SE, 67056 Ludwigshafen am Rhein (DE)
(72) Inventor: WEINGARTEN, Melanie, 67056 Ludwigshafen (DE); IFFLAND, Gabriele, 67056 Ludwigshafen (DE); SA GOMES, Pedro, 67056 Ludwigshafen (DE); PUHL, Michael, 67056 Ludwigshafen (DE); SIEGEL, Wolfgang, 67056 Ludwigshafen (DE); GARELLA, Linda, 67056 Ludwigshafen (DE); COOPER, Bryan, 79664 Wehr (DE); MUELLER, Patric, 67056 Ludwigshafen (DE); ZELDER; Oskar, 67056 Ludwigshafen (DE); SCHRÖDER, Hartwig, 67056 Ludwigshafen (DE)
(74) Representative: BASF IP Association

(57) **Abstract**

The present invention relates to an improved process for isolating aliphatic, mono- or polycyclic small molecules, in particular, Cycloclavine or Cornexistin and/or Hydroxycornexistin, from the aqueous fermentation broth of a eukaryotic microorganism. Said process comprises contacting a liquid phase of said fermentation broth containing said active ingredient with a suitable adsorbent, desorbing said active ingredient from said adsorbent, optionally performing a second chromatographic purification step, and isolating the active ingredient in substantially pure form. The thus obtained purified small molecules may be further used in biocidal, i.p. insecticidal or herbicidal compositions.

## Description

The present invention relates to an improved process for isolating aliphatic, mono- or polycyclic small molecules, in particular, Cycloclavine or Cornexistin and/or Hydroxycornexistin, from the aqueous fermentation broth of an eukaryotic microorganism. Said process comprises contacting a liquid phase of said fermentation broth containing said active ingredient with a suitable adsorbent, desorbing said active ingredient from said adsorbent, optionally performing a second chromatographic purification step, and isolating the active ingredient in substantially pure form. The thus obtained purified small molecules may be further used in biocidal, i.p. insecticidal or herbicidal compositions.

### Background of the invention

Biocidally active compounds, like the insecticide Cycloclavine or the herbicides Cornexistin and Hydroxycornexistin are compounds which may be isolated from naturally occurring or genetically modified sources. For that purpose, corresponding microorganisms are cultivated and the desired small molecule, together with a plurality of other metabolic by-products, often accumulates in the fermentation broth.

Stauffacher et al., Tetrahedron 1969, 25, 5879 isolated Cycloclavine 1, an ergot alkaloid from the seeds of Kenyan morning glory (*Ipomoea hildebrandtii* VATKE)*.* It was called cycloclavine due to its' unique structure containing one ring more, a cyclopropyl ring, than all up to that date known clavines.

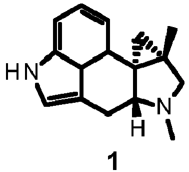

Cycloclavine was furthermore identified to be produced by different *Aspergillus* and *Argyrea* fungi (Chao, J.-M. et al. Phytochemistry 1973, 12, 2435. Furuta T., et al. Agric. Biol. Chem. 1982, 46, 1921; Parenicova, L. et al. Appl. Environ. Microbiology 2001, 67, 521.

Cycloclavine production by applying different *Saccharomyces cerevisiae* strains as producing organism are reported in WO2014/030096.

Cornexistin (2) and Hydroxycornexistin (3) 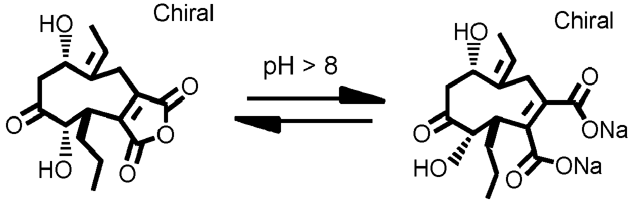 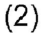 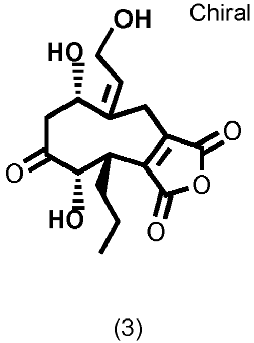 are natural products derived from the fungus *Paecilomyces divaricatus* 16891 formerly known as *Paecilomyces variotii* SANK21086*.* Both, Cornexistin and Hydroxycornexistin, are highly potent herbicides that have the unique quality of being harmless to corn plants. Because of this quality, both molecules have attracted research interest. Their interesting nine-membered ring structure contains a maleic anhydride and a key exocyclic ethylidene; this structure also indicates membership in the nonadride family activity and its highly functionalized structure. As part of these discovery efforts, the Sankyo Corporation discovered Cornexistin during the screening of biological extracts for herbicidal use (JP2256602). Cornexistin showed good activity as a herbicide as well as relative inactivity towards corn plants. Sankyo's characterization showed this fungal natural product to be a member of the nonadride family, a group of natural products known for their interesting structural characteristics including a central nine-membered ring, fused maleic anhydrides and pendant alkyl chains.

Cornexistin and Hydroxycornexistin have been synthesized by chemical synthesis only as diastereomeres (Org. Biomol. Chem. , 2008, 6, 4012-4025). Nine-membered carbocyclic structures in general are rare in nature and their synthesis as well as the genes involved in the synthesis are still unknown and not described. Isolation of cornexistin from the cultures of *Paecilomyces* species originally identified as *Paecilomyces variotii* SANK21086 was published as early as 1989 by the Sankyo research group JP2256602).

Later work from the DOW Elanco group described identification of hydroxycornexistin also produced in Paecilomyces variotii SANK 21086 (US 5,424,278).

US 2014/141440 relates to the production of Cornexistin and Hydroxycornexistin. It provides polynucleotides encoding polypeptides involved in the biosynthesis of Cornexistin and Hydroxycornexistin as well as vectors and recombinant microorganisms comprising such polynucleotides. Also provided are methods for the production of Cornexistin and Hydroxycornexistin, using such polynucleotides.

In the prior art, different attempts have been reported in order to isolate in sufficient yield and purity such microbially produced small molecules.

For example, US 4,897,104 describes several approaches for isolating the phytotoxin Cornexistin from the fermentation broth of *Paecilomyces variotii* Bainier*,* strain SANK 21086. According to one approach of US 4,897,104, the liquid phase of the fermentation broth is contacted with the adsorbent Diaion HP 20 in order to adsorb Cornexistin. Said adsorbent was eluted with aqueous acetone, the eluate was evaporated, the thus obtained crude product was redissolved in water, acidified to a pH-value of 2.5 and afterwards extracted in several steps with different solvents. Afterwards, the product was again dried, dissolved in a mixture of ethyl acetate and chloroform and, in a final chromatographic step, purified by applying Sephadex LH-20 in order to obtain Cornexistin in a purity of merely about 70 %. Other preferred approaches disclosed in US 4,897,104 describe the direct acidification of the cell-free fermentation product, followed by multiple extraction steps and, finally, by applying a Sephadex LH-20 chromatography, optionally followed by purification by HPLC using a reverse phase column in order to obtain a product of about 90 % purity.

Nakajima et al. J. Antibiotics, 1991, 44, 10, 1065-1β72 describe the isolation of Cornexistin from the culture filtrate of *Paecilomyces variotii* Bainier, strain SANK 21086 by applying a method comprising multiple extraction steps with ethyl acetate at pH 8 or pH 2.5, washing with saturated sodium chloride, in vacuo concentration to dryness, dissolving the oily substance in methylene chloride, followed by re- extraction with sodium dihydrogen phosphate solution, and followed by re-extraction with methylene chloride after adjustment of pH to 2.1. The extract was the washed with sodium dihydrogen phosphate solution and saturated NaCl solution and finally concentrated to dryness the obtained oily substance was then chromatographed over Sephadex LH-20 and eluted with methylene chloride/ethyl acetate (1:1).

US 5,425,278 describes the isolation of the herbicide Hydroxcornexistin which is also produced by *P. variotii* Bainier SANK 21086 . In order to isolate Hydroxycornexistin, a different isolation strategy was followed. In particular, the liquid phase of the fermentation broth in a first step is adjusted to pH12 and extracted with two volumes of ethyl acetate. While said ethyl acetate phase is discarded, the aqueous phase is acidified to a pH of 2. Afterwards, the extraction was repeated with ethyl acetate. From said ethyl acetate phase, a dark oil containing Hydroxycornexistin is obtained which is further purified by applying C₁₈-RP HPLC (see also Fields et al, J. Nat. Prod., 1996, 59, 698-700).

Neither US 4,897,104 nor US 5,425,278 disclose or suggest a process which allows the simultaneous isolation of Cornexistin and Hydroxycornexistin from the fermentation broth of a microorganism producing said two herbicides.

WO2014/030096 claims the isolation of Cycloclavine either by applying a liquid/liquid extraction step with ethyl acetate pH 10; or by applying a silica based solid phase extraction step at pH 3 or pH10.

The prior art does not disclose isolation methods suitable for large-scale production of small molecules like Cycloclavine, Cornexistin and Hydroxycornexistin as containe din the complex fermentation broth of a microorganism.

Therefore, a first object of the present invention is the provision of a process for isolating an aliphatic, mono- or polycyclic small molecule from the aqueous fermentation broth of an eukaryotic microorganism.

Another, more particular object of the invention is the provision of a process for isolating Cycloclavine from the aqueous fermentation broth of a eukaryotic microorganism, in particular the fermentation broth of the species *Saccharomyces cerevisiae.*

According to another more particular object, the present invention also relates to a process for the isolation of Cornexistin and/or Hydroxycornexistin, in particular the simultaneous isolation of Cornexistin and Hydroxycornexistin from the aqueous fermentation broth of a eukaryotic microorganism, in particular a microorganism of the species *Paecilomyces divaricatus.*

### Summary of the invention

The above-mentioned problems, surprisingly, could be solved by the provision of a simple and effective isolation process as defined in the attached claims. Said process is based on the decisive selection of a first process step which allows applying the complex liquid supernatant of a microbial fermentation broth to a suitable solid absorbent under conditions which allow the absorption of the desired active ingredients and the subsequent desorbing and enrichment of the active ingredient by applying a suitable organic eluent and optionally further purifying the obtained eluate in another solid phase-based separation step.

The processes of the present invention are simple, effective, and avoid multiple extraction steps which disfavor the large-scale industrial isolation of the intended active ingredient.

### Description of Figures

Figure 1a shows the HPLC chromatogram of the Cycloclavine containing liquid phase of a fermentation broth to be applied in the subsequent step of adsorption chromatography.
Figure 1b shows the HPLC analysis of Cycloclavine purified according to the present invention.
Figure 2a shows the accumulation of Cornexistin throughout a 200 L scale fermentation run.
Figure 2b shows the HPLC chromatogram of the liquid phase of the fermentation broth illustrating the formation of Cornexistin and Hydroxycornexistin.
Figure 3 shows the accumulation of Cornexistin throughout a fermentation run in the 2.000 L scale.
Figure 4 illustrates the distribution of Cornexistin and Hydroxycornexistin in different work-up stages of the fermentation broth.
Figure 5a shows the HPLC analysis of Cornexistin and Hydroxycornexistin in combined filtrates at pH 2.5 prior to adsorption chromatography.
Figure 5b shows the HPLC analysis of Cornexistin and Hydroxycornexistin in the combined eluates after adsorption chromatography.
Figure 5c shows the HPLC analysis of Cornexistin and Hydroxycornexistin in the combined eluates after adsorption chromatography and after removal of ethyl acetate.
Figure 6 shows the HPLC analysis of two fractions containing Cornexistin (Fig. 6a)) and Hydroxycornexistin (Fig. 6b)) as obtained by silica gel chromatography.
Figure 7 shows the corresponding HPLC analysis of Cornexistin (Fig. 7a)) and Hydroxycornexistin (Fig. 7b)) as obtained by preparative RP-HPLC.

### Detailed description

### a) General definitions

A "small molecule" according to the present invention refers to natural or synthetic, in particular natural compounds having a molar mass below about 2.000, more particularly below 1.500, and preferably below 1.000, and at least 100, more particularly at least 150 and preferably at least 200g/mole.

An "aqueous fermentation broth" refers to a liquid phase as obtained by running a fermentation process of a microorganism, in particular a eukaryotic microorganism, like a yeast strain. The broths may typically contain insolubles, like cells of the microorganism, cell debris thereof, remaining constituents of the original fermentation medium as well as products resulting from the fermentation process, in particular metabolic products produced by said microorganism.

An "aliphatic polymeric resin" as applied according to the present invention consists of or comprises a majority of (molar excess of) aliphatic monomeric constituents. Preferred aliphatic monomers are acrylic acid and methacrylic acid monomers in the form of free acids or ester derivatives thereof.

An "aromatic polymeric resin" as applied according to the present invention consists of or comprises a majority of (molar excess of) aromatic monomeric constituents. Preferred aliphatic monomers are styrene monomers.

An "aliphatic and aromatic polymeric resin" as applied according to the present invention consists of or comprises a majority of (molar excess of) aromatic and aliphatic monomeric constituents. The molar ratio of aliphatic and aromatic monomers may vary depending on the respective separation problem.

"Purification" and "Isolation" both encompass any degree of partial enrichment as well as the complete purification of the intended compound of interest.

The term "pure" encompasses analytically pure (i.e. contaminating constituents are analytically non-detectable) as well as a degree of purity of at least 70, 75, 80, 95, 90, 91, 92, 93, 94, 95, 96, 97, 98, 99, 99.5, 99.6, 99.7, 99.8 or 99.9 % by weight.

Compounds isolated according to the present invention may be isolated in the form of stereoisomerically pure compounds or as mixture of two or more stereoisomers/diastereomeres.

Compounds isolated according to the present invention and carrying one or more carboxylic residues may be isolated in the form of the free acid, an intra- or intermolecular anhydride form, as alkaline (Na or K) or earth alkaline (Ca, Mg) metal salt or alkyl ester (i.p. lower (preferably C₁-C₄) alkyl ester.

Compounds isolated according to the present invention and carrying one or more amino groups may be isolated as uncharged compounds or also in the form or corresponding ammonium salts.

### b) Preferred embodiments

The present invention relates, in particular, to the following preferred embodiments:
1. A process for isolating at least one, in particular one or two aliphatic, mono- or polycyclic (condensed or non-condensed, carbo- or hetrerocyclic rings) in particular mono- or bicyclic, small molecules (also designated "active ingredient"), like Cycloclavine, Cornexistin or Hydroxycornexistin, from the aqueous fermentation broth of a eukaryotic microorganism producing at least one of said small molecules to be isolated, comprising the steps of
   a) performing a solid/liquid separation step, like centrifugation or filtration or combinations thereof, in order to remove biomass (i.p. insoluble constituents, like complete cells or cell debris) from the fermentation broth;
   b) contacting the obtained liquid phase of step a) with a first porous adsorbent, which adsorbs said small molecule,
   c) desorbing the small molecule by eluting the adsorbent with an organic solvent, and
   d) isolating the active ingredient from the eluate.
   The process may be performed continuously, semi-batch-wise or, preferably batch-wise.
   If appropriate, individual steps may be repeated once or several times in order to improve the result of product isolation. Preferably no repetition of individual steps is required.
   If appropriate, before perfoming the next step the liquid phase to be applied may be subjected to a concentration step, for example, by removing a solvent constituent by evaporation.
   After biomass separation an additional filtration step may be applied in order to further clarify the obtained liquid phase.
   If appropriate, the pH of the liquid phase as obtained by step a) may be modified (increased or decreased) in order to improve the separation efficiency of the subsequent purification steps. Any organic or inorganic acid or base may be applied. Preferably the pH is decreased by adding hydrochloric acid or sulfuric acid. Preferably the pH is increased by adding sodium hydroxide or calcium hydroxide.
2. The process according to embodiment 1, wherein said small molecule is a biocide, in particular herbicide, like Cycloclavine, Cornexistin or Hydroxycornexistin.
3. The process of embodiment 1 or 2, wherein the first porous adsorbent as used in step b) is an aliphatic and/or aromatic polymeric adsorbent, a basic adsorbent, an acidic adsorbent or charcoal, preferably an aliphatic or aromatic polymeric adsorbent, in particular an aliphatic or aromatic polymeric resin; or an adsorbent containing both aliphatic and aromatic monomeric constituents.
4. The process of embodiment 3, wherein the first porous adsorbent is a polyacrylate-based or a polyaromatic-based (i.p. polystyrene-based) adsorbent or a polymeric adsorbent composed of both acrylic and aromatic monomeric constituents , i.e. an acrylic and styrene monomer based polymeric adsorbent.
5. The process of embodiment 4, wherein said adsorbent is characterized by at least one of the following parameters:
   a) polarity of matrix in combination with the polarity of the solvent corresponding to the polarity of the compound to be purified;
   b) pore diameter in the range of 55 to 550 Angstrom, preferably 100 to 1550 and particularly preferred 400 - 550 Angstrom;
   c) surface area in the range of 300 to 1.000, in particular 500 to 900, particularly preferred 600 to 800 m²/g
   d) porosity of at least 0.50 ml/ml, particularly preferred 0.5 - 0.6 ml/ml
   e) particle size in the range of 0,2 to 2,5 mm, preferably 0,25 to 1, particularly preferred 0,3 to 0,5mm
   In particular, the adsorbent is characterized by above feature b) or c) or d) or e); More particularly it is characterized by a combination of feature b) and c), b) and d) or b) and e). Most preferably, it is characterized by features b), c), d) and e).
6. The process of embodiment 4 or 5, wherein
   a) said aliphatic or aromatic polymeric adsorbent is Amberlite ®XAD® 7 HP, Amberlite ®XAD®1180 or Treversorb ADS700; preferably Amberlite ®XAD® 7 HP.
   b) said basic adsorbent is selected from Ambersep 900 OH, Amberlite IRA 900 CI or Lewatit MP 62;
   c) said acidic adsorbent is selected from Amberlite ®IRC 86, Amberlite 252H or Lewatit ® K2621; or
   d) said charcoal is selected from Norit C Gran and CAL ® Chemviron.
7. The process of one of the preceding embodiments, wherein the fermentation broth or the liquid phase of step a) is subjected to a pH adjustment.
8. The process of one of the preceding embodiments, wherein the eluate of step c) is contacted with a second adsorbent which adsorbs said small molecule.
9. The process of embodiment 8, wherein said second adsorbent is silica gel.
10. The process of embodiment 8 or 9, wherein the small molecule is desorbed by eluting the second adsorbent with an organic solvent, preferably ethyl acetate.
11. The process of one of the preceding embodiments, wherein said small molecule is selected from Cycloclavine or Cornexistin or Hydroxycornexistin.
12. The process of one of embodiments 1 to 11 for isolating Cycloclavine from the aqueous fermentation broth of a eukaryotic, natural or genetically modified Cycloclavine producing microorganism, comprising
   a) performing a solid/liquid separation step in order to remove biomass from the fermentation broth;
   b) contacting the obtained liquid phase of step a) with a first porous adsorbent, which adsorbs Cycloclavine,
   c) desorbing the Cycloclavine by eluting the adsorbent with an organic solvent, and
   d) isolating the active ingredient from the eluate.
13. The process embodiment 12, wherein the eukaryotic microorganism is a microorganism of the genus *Saccharomyces,* in particular *S. cerevisiae.*
14. The process of anyone of embodiments 12 or 13, wherein the fermentation broth or the liquid phase of step a) is alkalized, preferably adjusted to a pH in the range of 8 to 11, preferably 9 to 10.
15. The process of anyone of the embodiments 12 to 14, wherein the first porous adsorbent as used in step b) is an aliphatic polymeric adsorbent, in particular a polyacrylate-based adsorbent and preferably Amberlite ®XAD® 7 HP.
16. The process of anyone of the embodiments 12 to 15, wherein Cycloclavine is desorbed from the adsorbent by washing with a polar organic solvent, in particular, ethyl acetate, MTBE, THF, acetone or mixtures of at least two of them, preferably ethyl acetate.
17. The process of anyone of the embodiments 12 to 16, wherein the eluate from the first adsorbent or a crude product obtained from said eluate by removal of the solvent and redissolving in a more non-polar second solvent, in particular mixtures of MTBE and cyclohexane, preferably in a ratio (vol/vol) of 1 : 1, 2 : 1, or 1 : 2, is contacted with a second adsorbent, in particular silica gel, which adsorbs Cycloclavine .
18. The process of embodiment 17, wherein Cycloclavine is desorbed by washing the second adsorbent with an organic solvent, in particular MTBE or a MTBE gradient.
19. The process of one of the embodiments 1 to 11 for isolating Cornexistin and/or Hydroxycornexistin from the aqueous fermentation broth of a eukaryotic, natural or genetically modified Cornexistin and/or Hydroxycornexistin producing microorganism, comprising
   a) performing a solid/liquid separation step in order to remove biomass from the fermentation broth;
   b) contacting the obtained liquid phase of step a) with a first porous adsorbent, which adsorbs Cornexistin and/or Hydroxycornexistin,
   c) desorbing the Cornexistin and/or Hydroxycornexistin by eluting the adsorbent with an organic solvent, and
   d) isolating the active ingredient from the eluate.
20. The process of embodiment 19, wherein the eukaryotic microorganism is a microorganism of the genus *Paecilomyces,* in particular *P. divaricatus.*
21. The process of anyone of embodiments 19 or 20, wherein the fermentation broth or the liquid phase of step a) is acidified, preferably adjusted to a pH in the range of 1 to 4, preferably 2 to 2.5.
22. The process of anyone of the embodiments 19 to 21, wherein the first porous adsorbent as used in step b) is an aliphatic polymeric adsorbent, in particular a polyacrylate-based adsorbent and preferably Amberlite ®XAD® 7HP or Treversorb ADS700; or is an aromatic polymeric resin, preferably a polystyrene-based resin, like Amberlite ®XAD®1180
23. The process of anyone of the embodiments 19 to 22, wherein Cornexistin and/or Hydroxycornexistin is desorbed from the adsorbent by washing with a polar organic solvent, in particular, ethyl acetate, MTBE, THF, acetone or mixtures thereof, preferably ethyl acetate.
24. The process of anyone of the embodiments 19 to 23, wherein the eluate from the first adsorbent or a crude product obtained from said eluate by removal of the solvent redissolving in a more non-polar second solvent, in particular mixtures of MTBE and cyclohexane, preferably in a ratio (vol/vol) of 1 : 1, 2 : 1, or 1 : 2, is contacted with a second adsorbent, in particular silica gel, which adsorbs Cornexistin and/or Hydroxycornexistin.
25. The process of embodiment 23 or 24, wherein Cornexistin and Hydroxycornexistin are desorbed separately by washing the second adsorbent with an organic solvent, in particular by applying a solvent gradient of petroleum ether: acetone, in particular in a range of about 10:1, to 1:1, preferably comprising the gradient steps 10:1, 5:1, 4:1, 5:2, 2:1, and 1:1.
26. The process of embodiment 25, wherein Cornexistin and Hydroxycornexistin are further purified by RP PHLC, in particular by applying a C18 reversed phase column with acetonitrile-water-trifluoroacetic acid or acetonitrile-water-formic acid as eluent.
27. The process of embodiment 26, wherein Cornexistin is purified by applying the following gradient: 70 % water (+0,1% TFA) / 30% MeCN (+0,05% TFA), 80 % water (+0,1% TFA) / 20% MeCN (+0,1% TFA), and 50 % water (+0,1% TFA) / 50% MeCN (+0,1% TFA);
28. The process of eembodiment 26, wherein Hydroxycornexistin is purified by applying the following gradient: 90 % water (+0,1% TFA) / 10% MeCN (+0,05% TFA), 90 % water (+0,1% TFA) / 10% MeCN (+0,1% TFA), 60 % water (+0,1% TFA) / 40% MeCN (+0,1% TFA)).
29. A small molecule, in particular Cycloclavin, Cornexistin and/or Hydroxycornexistin or a product containing at least one small molecule, obtainable by a process of anyone of the preceding embodiments.
30. A biocidal composition comprising at least one compound of embodiment 29, optionally in combination with a further biocidally active constituent.

The present invention will be explained in more detail by exemplifying a particular separation process for isolating Cycloclavine and another process of co-isolating Cornexistin and Hydroxycornexistin.

### c) Cultivation of Microorganisms

The invention is based on the efficient isolation of particular biocidally active substances from the fermentation broth of cultivated microorganisms, in particular fungal microorganisms.

A natural or genetically modified microorganism is cultured, optionally the expression of genes required for the production of the active substance(s) is induced and the thus produced active substance may be isolated from the fermentation broth. Fermentation may be performed in laboratory or industrial scale.

In general the cultivation of microorganisms can be performed under conditions conventionally employed for the cultivation of micororganisms, i.p. fungi, as for example fungi imperfecti. Preferably a liquid culture, and desirably with shaking or stirring and aeration may be performed. The nutrient medium used for the cultivation is completely conventional and contains such constituents as are commonly used in the cultivation of fungi imperfecti. Specifically, the medium should preferably contain an assimilable carbon source, suitable examples of which include glucose, maltose, sucrose, mannitol, molasses, glycerol, dextrin, starch (a particularly useful starch source for this microorganism, as for most fungi imperfecti, is fresh potato), soybean oil and cottonseed oil; an assimilable nitrogen source, suitable examples of which include soybean meal, peanut meal, cottonseed meal, Fermamine, fish meal, corn steep liquor, peptone, meat extract, yeast (e.g. pressed yeast), yeast extract, sodium nitrate, ammonium nitrate or ammonium sulfate; and one or more inorganic salts, such as sodium chloride, phosphates, calcium carbonate and, if required, trace metal salts. Where cultivation is effected in a liquid medium, it is generally desirable to incorporate an anti-foaming agent (for example silicone oil, vegetable oil or a suitable surfactant) in the medium.

The cultivation is suitably performed in a medium at a pH which may range from weakly acidic to substantially neutral and at a temperature of from 20 to 30, more preferably about 24 °C.

In more general terms, the microorganisms applied according to the invention can be cultured continuously or discontinuously in the batch method or in the fed-batch method or repeated fed-batch method. A summary of known cultivation methods can be found in the textbook by Chmiel (Bioprozesstechnik 1. Einführung in die Bioverfahrenstechnik [Bioprocess technology 1. Introduction to bioprocess technology] (Gustav Fischer Verlag, Stuttgart, 1991)) or in the textbook by Storhas (Bioreaktoren und periphere Einrichtungen [Bioreactors and peripheral equipment] (Vieweg Verlag, Braunschweig/Wiesbaden, 1994)).

The culture medium to be used must suitably meet the requirements of the respective strains. Descriptions of culture media for various microorganisms are given in the manual "Manual of Methods for General Bacteriology" of the American Society for Bacteriology (Washington D. C., USA, 1981).

These media usable according to the invention usually comprise one or more carbon sources, nitrogen sources, inorganic salts, vitamins and/or trace elements.

Particular carbon sources are sugars, such as mono-, di- or polysaccharides. Very good carbon sources are for example glucose, fructose, mannose, galactose, ribose, sorbose, ribulose, lactose, maltose, sucrose, raffinose, starch or cellulose. Sugars can also be added to the media via complex compounds, such as molasses, or other by-products of sugar refining. It can also be advantageous to add mixtures of different carbon sources. Other possible carbon sources are oils and fats, for example soybean oil, sunflower oil, peanut oil and coconut oil, fatty acids, for example palmitic acid, stearic acid or linoleic acid, alcohols, for example glycerol, methanol or ethanol and organic acids, for example acetic acid or lactic acid.

Nitrogen sources are usually organic or inorganic nitrogen compounds or materials that contain these compounds. Examples of nitrogen sources comprise ammonia gas or ammonium salts, such as ammonium sulfate, ammonium chloride, ammonium phosphate, ammonium carbonate or ammonium nitrate, nitrates, urea, amino acids or complex nitrogen sources, such as corn-steep liquor, soya flour, soya protein, yeast extract, meat extract and others. The nitrogen sources can be used alone or as a mixture.

Inorganic salt compounds that can be present in the media comprise the chloride, phosphorus or sulfate salts of calcium, magnesium, sodium, cobalt, molybdenum, potassium, manganese, zinc, copper and iron.

Inorganic sulfur-containing compounds, for example sulfates, sulfites, dithionites, tetrathionates, thiosulfates, sulfides, as well as organic sulfur compounds, such as mercaptans and thiols, can be used as the sulfur source.

Phosphoric acid, potassium dihydrogen phosphate or dipotassium hydrogen phosphate or the corresponding sodium-containing salts can be used as the phosphorus source.

Chelating agents can be added to the medium, in order to keep the metal ions in solution. Especially suitable chelating agents comprise dihydroxyphenols, such as catechol or protocatechuate, or organic acids, such as citric acid.

The fermentation media used according to the invention usually also contain other growth factors, such as vitamins or growth promoters, which include for example biotin, riboflavin, thiamine, folic acid, nicotinic acid, pantothenate and pyridoxine. Growth factors and salts often originate from the components of complex media, such as yeast extract, molasses, corn-steep liquor and the like. Moreover, suitable precursors can be added to the culture medium. The exact composition of the compounds in the medium is strongly dependent on the respective experiment and is decided for each specific case individually. Information on media optimization can be found in the textbook "Applied Microbiol. Physiology, A Practical Approach" (Ed. P.M. Rhodes, P.F. Stanbury, IRL Press (1997) p. 53-73, ISBN 0 19 963577 3). Growth media can also be obtained from commercial suppliers.

All components of the medium are sterilized, either by heat (20 min at 1.5 bar and 121°C) or by sterile filtration. The components can either be sterilized together or separately if necessary. All components of the medium can be present at the start of culture or can be added either continuously or batch-wise.

The culture temperature is normally between 15°C and 45°C, preferably 20°C to 30°C and can be varied or kept constant during the experiment. The pH of the medium should be in the range from 5 to 8.5, preferably around 7.0. The pH for growing can be controlled during growing by adding basic compounds such as sodium hydroxide, potassium hydroxide, ammonia or ammonia water or acid compounds such as phosphoric acid or sulfuric acid. Antifoaming agents, for example fatty acid polyglycol esters, can be used for controlling foaming. To maintain aerobic conditions, oxygen or oxygen-containing gas mixtures, for example ambient air, are fed into the culture. The culture is continued until a maximum of the desired product has formed. This target is normally reached within 10 hours to 300 hours.

The fermentation broth is then processed further. Depending on requirements, the biomass can be removed from the fermentation broth completely or partially by separation techniques, for example centrifugation, filtration, decanting or a combination of these methods or can be left in it completely.

Normally the active ingredients of interest are secreted in the culture medium. If this is not the case, the cells can also be lysed and the product can be obtained from the lysate by known methods for isolation of proteins. The cells can optionally be disrupted with high-frequency ultrasound, high pressure, for example in a French press, by osmolysis, by the action of detergents, lytic enzymes or organic solvents, by means of homogenizers or by a combination of several of the aforementioned methods.

### d) Isolation of Cycloclavine

The fermentative production of Cycloclavine as such is known in the art and may be performed by applying a genetically engineered *S. cerevisiae* strain produced an cultivated as described for example in WO 2014/030096.

In order to remove biomass the fermentation broth containing the Cycloclavine standard techniques like centrifugation of filtration may be employed. For example, the broth may be centrifuged to separate cells and cell debris, optionally followed by a filtration step in order to further clarify the liquid phase.

In a next step the adsorbent, in particular of the type Amberlite ®XAD®7, is prepared, for example by providing a suitable vessel, for example a glass column, containing a bead of suitable dimension and adsorbing capacity of the adsorbent. Before applying the broth the adsorbent may be washed with solvent (like water) in order to remove any impurity from the adsorbent material.

The Cycloclavine supernatant as obtained from the fermentation broth may be pre-treated by alkalization, for example to adjust a pH in the range of 8.5 to 10.5, using aqueous concentrated NaOH.

Then said supernatant is applied to the resin. Subsequently, after loading is completed, the resin was washed with distilled water in order to remove any non-adsorbed material.

In a next step the elution of Cycloclavine was performed by means of a suitable organic eluent. Preferably ethyl acetate is applied. The eluent may be applied in concentrated form or applied in the form of a (continuously or stepwise increasing) gradient. Preferably pure ethyl acetate is applied. Fractions of the eluate are taken and are analyzed for the presence of Cycloclavine.

In a next step the combined fractions enriched for Cycloclavine but still containing impurities are subjected to a further chromatographic separation step.

For that purpose a second chromatography vessel, such as a glass column may be provided containing a bead of suitable dimension and capacity of silica gel, pre-washed for example with the same solvent system as the liquid Cycloclavine phase to be applied thereon. The Cycloclavine containing phase as obtained above may be applied directly onto said silica gel adsorbent. Cycloclavine is adsorbed to said solid phase and may be eluted there form by applying a stronger polar organic solvent. Preferably MTBE (methyl t-butyl ether) is applied. If desirable and in order to further improve separation of contaminants it may be possible to apply a solvent gradient (stepwise or continuous), for example form an apolar organic solvent to an polar organic solvent, like a gradient from 100% cyclohexanes to 100% MTBE.

Fractions of the eluate are taken and analyzed for Cycloclavine. Combined fractions may be concentrated by evaporating the organic solvent. If necessary a second silica gel purification step may be performed in order to further increase purity of the Cycloclavine product.

### e) Isolation of Cornexistin and Hydroxycornexistin

The fermentative production of Cornexistin and Hydroxycornexistin as such is known in the art and may be performed by applying known techniques and known microorganisms which produce Cornexistin and/or Hydroxycornexistin, preferably both Cornexistin and Hydroxycornexistin.

For example the fungal strain *Paecilomyces divaricatus* LU16891 may be applied.

In order to remove biomass the fermentation broth containing the Cornexistin and Hydroxycornexistin standard techniques like centrifugation of filtration may be employed. In this case a filtration step, more particular a conventional membrane filter system, may be applied.

The thus obtained filtrate and optional washing liquids from a final washing step of the filter residue are used as starting materials for the subsequent adsorption chromatograthy.

In a next step the adsorbent, in particular of the type Amberlite ®XAD®7, is prepared, for example by providing a suitable vessel, for example a glass column, containing a bead of suitable dimension and adsorbing capacity of the adsorbent. Before applying the broth the adsorbent may be washed with solvent (like water) in order to remove any impurity from the adsorbent material.

The filtrate containing Cornexistin and Hydroxycornexistin resulting from the membrane filtration in a first step acidified, to a pH in the range of 2 to 3, preferably about pH 2.5 by adding sulfuric acid and is then loaded onto the column. After completeing the loading a solvent, like distilled water may be used to wash the column in order to remove non.bound constituents.

In next step the elution of Cornexistin and Hydroxycornexistin was performed by means of a suitable organic eluent. Preferably ethyl acetate is applied. The eluent may be applied in concentrated for or applied in the form of a (continuously or stepwise increasing) gradient. Preferably pure ethyl acetate is applied. Fractions of the eluate are taken and are analyzed for the presence of Cornexistin and Hydroxycornexistin.

The combined Cornexistin and Hydroxycornexistin containing material was then further purified by silica gel chromatography.

The ethyl acetate solution may be applied as such or concentrated under reduced pressure in order to reduce the total volume of the sample to be applied onto a silica gel column. For that purpose a second vessel, for example a glass column, may be provided containing a bead of suitable dimension and adsorbing capacity of silica gel, pre-washed for example with the same solvent system as the liquid Cornexistin and Hydroxycornexistin containing phase to be applied thereon. The Cornexistin and Hydroxycornexistin containing phase as obtained above may be applied directly onto said silica gel adsorbent.

Silica gel column purification by which Cornexistin and Hydroxycornexistin are substantially separated from each other may be performed by applying a suitable solvent gradient (stepwise our continuous). Particularly preferred is a gradient system of petroleum ether: acetone. More preferably a stepwise gradient of petroleum ether: acetone of the steps 10:1, 5:1, 4:1, 5:2, 2:1, 1:1 may be applied in order to separate and enrich Cornexistin and Hydroxycornexistin.

The two components may the be further purified by applying a C₁₈-RP HPLC purification step

For each of the two constituents a water/ MeCN gradient system may be applied (optionally supplemented with 0.1% v/v TFA) in order to isolate the two constituents in high purity.

Finally the solvent may be removed be evaporation.

### Experimental Part

### General Methods

### a) HPLC Analytics Cycloclavine:

| | | |
|---|---|---|
| Apparatus | : | Agilent Series 1100 |
| Column | : | Zorbax Eclipse XDB-C18 1,8µm 50*4,6mm by Agilent® |
| Eluent | : | -A: Water 0,1 Vol.-% H₃PO₄ |
| | | -B: Acetonitrile |

| time in min | %B | Flow rate [mL/min] |
|---|---|---|
| 0,0 | 1 | 1,4 |
| 4,0 | 50 | 1,4 |
| 5,5 | 100 | 1,4 |
| 7,5 | 100 | 1,4 |
| 7,6 | 1 | 1,4 |

| | | |
|---|---|---|
| Detector | : | UV-Detector λ=210 nm, BW=4 nm |
| Flow rate | : | 1,4 ml/min |
| Injection | : | 5 µL |
| Temperature | : | 40°C |
| Duration | : | 9,5 min |
| Pressure | : | ca. 210 bar |

### b) HPLC Analytics Cornexistin/Hydroxycornexistin:

| | | |
|---|---|---|
| Apparatus | : | Agilent Series 1100 |
| Column | : | Zorbax Eclipse XDB-C18 1,8µm 50*4,6mm from Agilent® |
| Eluent | : | -A: Water with 0,1Vol% H₃PO₄ |
| | | -B: Acetonitrile |

| time in min | %B | Flow rate [mL/min] |
|---|---|---|
| 0,0 | 20 | 1,5 |
| 4,0 | 45 | 1,5 |
| 6,0 | 100 | 1,5 |
| 8,0 | 100 | 1,5 |
| 8,1 | 20 | 1,5 |

| | | |
|---|---|---|
| Detector | : | UV-Detector λ=210 nm, BW=4 nm |
| Flow rate | : | 1,5 ml/min |
| Injection | : | 5 µL |
| Temperature | : | 40°C |
| Time | : | 10 min |
| Pressure | : | ca. 220 bar |

### Example 1: Isolation of Cycloclavine 1

### Summary:

A successful downstream process for the fermentation broth containing the insecticide Cycloclavine 1 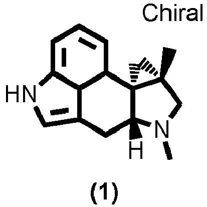 was developed.

A genetically modified strain of the yeast *Saccharomyces cerevisae* produced Cycloclavine. The purity of Cycloclavine is only 1.4 HPLC-a% (area %) in the broth. The active ingredient is exclusively found in the supernatant. Therefore, centrifugation and subsequent clear filtration were performed to remove the biomass. Then, a very efficient adsorption chromatography technique was employed for a solvent switch from water to ethyl acetate including a reduction of the solvent volume and a first very good purification. Amberlite ® XAD®7 turned out to be a resin of choice and Cycloclavine was obtained with a yield of up to 86% an excellent purity of up to 88 HPLC-a% (starting from 1.4 HPLC-a%). The crude material was then purified by silica gel chromatography resulting in a purity of 95 HPLC-a%.

### Example 1.1: Fermentation of Saccharomyces cerevisae - 12 L scale

A genetically modified strain of the yeast *Saccharomyces cerevisae* was applied for the fermentative production of Cycloclavine. Said recombinant strain having the internal designation EYS2325 has the following genotype:
MATα MAL2-8C SUC2 his3Δ 1 leu2-3_112 ura3-52 YERCΔ 8::AjEasC/KanMX YHRCΔ 14::ScPdi1/ ScFad1/BleR YMRWΔ 15::AjEasH/AjDmaW/HygRYN-RCΔ 9::AjEasH/AjEasC/AjEasE/Nat YORWΔ 17::AjDmaW/AfEasF YORWΔ 22::AjEasH/AjEasD YPRCt3::AjEasG/AjEasA YPRCΔ 15::AjEasE/AjEasC [ARS/CEN/URA3] [ARS/CEN/HIS3] [ARS/CEN/LEU2]

and was produced as described in WO 2014/030096, (see Examples 13 and 14 in WO 2014/030096) (the content of this document being incorporated by reference).

The fermentative production of Cycloclavine by applying EYS2325 was performed according to WO 2014/030096 (Examples 14).

In order to remove biomass the fermentation broth was centrifuged (Sorvall RC12BP), (beaker volume 2 L) for 30 minutes at 4.700 Rpm (7.500g), followed by a filtration step by applying a vacuum bottle with suction filter and filter pater Machery-Nagel, 126/70, Art.Nr. 400126/70/024, 10-15µm). The Cycloclavine content was determined by HPLC analytics.

Cycloclavine was produced in 168 h with a titer of ∼ 55 mg/L on a 12 L scale. In the fermentation broth the purity of Cycloclavine was 1.4 HPLC-a%.

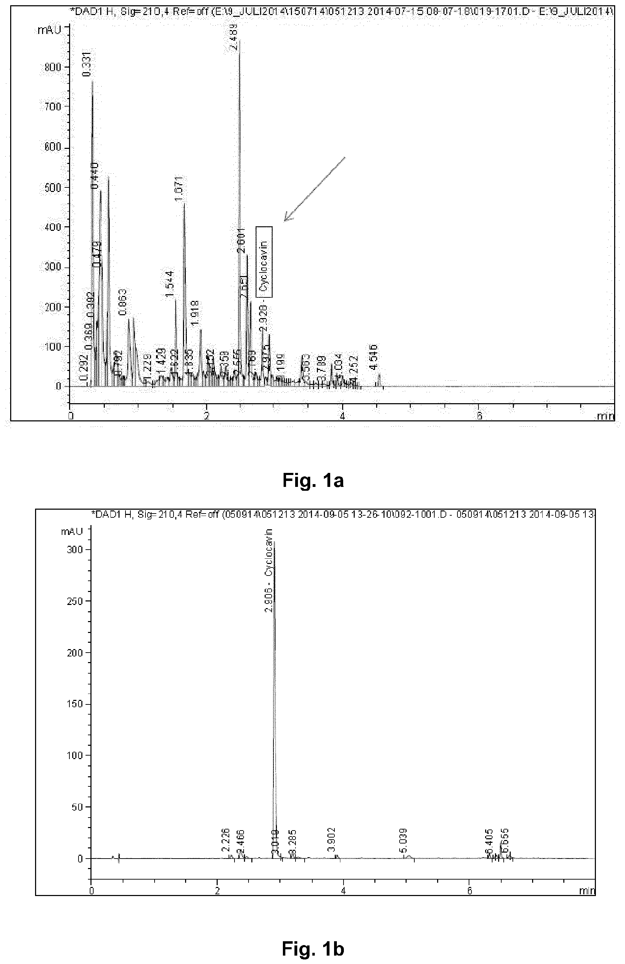

Figure 1a shows the HPLC chromatogram of the liquid phase of the fermentation broth to be applied in the next purification step (adsorption chromatography)

### Example 1.2: Adsorption chromatography of Cycloclavine broth on 5.68 L scale

70 mL of Amberlite ®XAD®7 were mixed with distilled water and were poured into a 90 mL glass column (diameter: 2.5 cm, height: 15 cm). The resin was washed with 2.3 L = 33.3 BV (BV = bed volume) distilled water. Then, the Cycloclavine supernatant (obtained by centrifugation of the fermentation broth and subsequent clearing filtration of the supernatant) was brought to pH 9.5 using 25% NaOH and applied to the resin. Subsequently, the resin was washed with distilled water and elution of the a.i. was performed using ethyl acetate.

The following fractions were taken and the following volumes and velocities were applied:

| **Task** | **Solvent** | **Amount** | | **Velocity** | **Fractions combined** | | |
|---|---|---|---|---|---|---|---|
| | | **L** | **BV** | **BV/h** | **#** | **BV** | **ml** |
| Loading | Cycloclavine supernatant | 5.68 | 81 | 5 | 1-8 | 10 | 700 |
| | | | | | 9 | 3 | 210 |
| Washing | dist. water | 1.4 | 20 | 5 | 10-13 | 3 | 210 |
| Elution | EtOAc | 1.75 | 15 | 4 | 14-25 | 1 | 70 |
| | | | | | 26 | 3 | 210 |

This corresponds to

| **Total amount** | **mg** | **181.4** |
|---|---|---|
| **Yield** | **%** | **77.2** |
| **Loss** | **%** | **0.1** |
| **Recovery rate** | **%** | **77.3** |

### Example 1.3: Final purification of Cycloclavine

2.1 g of crude combined Cycloclavine fractions (resulting from work-up of a total amount of supernatant = 8.24 L) were filtered over silica gel using MTBE. The 40 mL fractions taken were analyzed by DC (MTBE, R_{f} (Cycloclavine in cyclohexane:MTBE 1:1) ≈ 0.13, UV & anisaldehyde stain). Fractions 3-6 were combined and the solvent was removed completely under reduced pressure at 250 - 5 mbar and 30 °C to yield 1.11 g of brown syrup. With a titer of ∼ 55 mg/L in theory a much lower amount of 453 mg of Cycloclavine is expected. ¹³C-NMR revealed that Pluriol P 2000 (Polyethyleneglycoldiisopropylether, CAS 77698-35-2) is still in the sample as a major component (Pluriol P 2000 was used in the fermentation process as antifoam agent). A further silica gel column chromatography using cyclohexanes -> MTBE/cyclohexanes 1 : 1 as eluent could successfully remove Pluriol P 2000 (not visible of course by UV and by anisaldehyde stain). Working-up of fermentation broths devoid of Pluriol do not require a second silica gel purification step.

The purity was further increased by silica gel column chromatography from 89 to 95 HPLC-a%. 95 mg of Cycloclavine with 95% HPLC-a% and 80 wt% by quant. NMR were obtained as slightly brown solid.

After quant. NMR using 1,3,5-Trimethoxybenzene as internal standard another prep. TLC (Cyclohexane : MTBE = 2 : 1, R_{f}= 0.45) was performed with this material (56 mg received from quant. NMR after removal of solvent).

119 mL of Cycloclavine solution in EtOAc with 86 HPLC-a% purity and 183.55 mg/L which corresponds to 21.8 mg Cycloclavine pure was obtained. (see Fig. 1b)

### Example 2: Isolation of Cornexistin 2 and Hydroxycornexistin 3

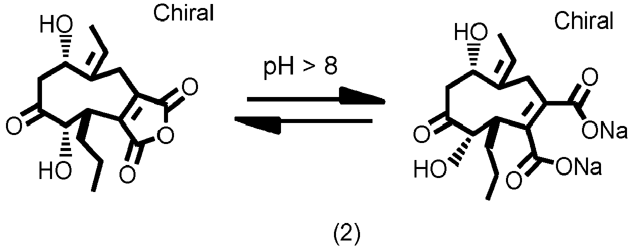

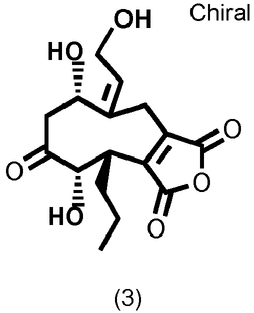

### Example 2.1: Fermentation of Paecilomyces divaricatus LU16891 and work-up for the production of Cornexistin and Hydroxycornexistin in 200L volume

The strain *Paecilomyces divaricatus* LU16891 (formerly known as *Paecilomyces variotii* SANK21086 as, for example, described in US 4,897,104 or US 5,424,278) was grown in a two-step fermentation.

### a) Pre-Culture:

Cells were stored as spores after growth on potato agar. From the spore cell bank a pre-culture was inoculated and grown in the following media designated S#5:

**Composition of pre-culture medium:**

| Raw material | Pre-culture medium S#5 concentration [g/L] |
|---|---|
| Polypeptone (Becton Dickinson) | 20 |
| Malt extract (Becton Dickinson) | 10 |
| Yeast extract (Becton Dickinson) | 10 |
| K₂HPO₄ (Roth Chemicals) | 1 |
| MgSO₄ x 7 H₂O (Roth Chemicals) | 0,5 |
| Glucose monohydrate | 20 |

8 x 2L-Erlenmeyer shaking flasks were filled with 400 mL S#5 Medium. They were each inoculated with 0,5ml of a frozen spore solution containing 3,1*10E8 spores/ml. The 2L shake flasks without baffles covered with Kimguard cover were incubated for 96h at 26°C with an RPM of 220 and an amplitude of 4cm.

### b) Main Culture:

**Composition of main culture medium:**

| Raw material | Main culture medium [g/L] |
|---|---|
| Potato flakes (Hanneforth) | 50 |
| Glycerol (technical garde 99% purity) | 50 |
| Pluriol P2000 | 0,04 |
| Urea | 4 |

### Preparation of main culture medium

Potato flakes, Glycerol and Pluriol were added to the fermenter and mixed with 170 L demineralized water and autoclaved for 60min at 123°C. Urea solution was prepared as follows: 920 g urea was dissolved in 4000g demineralized water and autoclaved at 121°C for 30min, the dissolved and sterile was added to the fermentation medium.

### Execution of main culture

The 3500 mL Medium of the seed culture were inoculated for 96h as described and were homogenized by Ultraturrax treatment and inoculated in the main culture medium:
The main culture was performed as follows: The fermenter setting were done as follows: The aeration was set to 25 L/min, the pH value of the medium was set to 6,1 by adding H₂SO₄ solution. The dissolved pO₂ value was controlled at 30% by controlling the stirring speed 3 disk plate stirrers were implemented in the fermenter, the lower stirrer speed was set to 200 rpm. During the fermentation the stirring speed increased to 350rpm. Glycerol concentration was kept above 10g/I throughout fermentation by adding amounts of glycerol continuously to the fermentation. After 402h the fermentation was terminated. pH was not controlled throughout fermentation. Samples were withdrawn and prepared for the analysis of glycerol and Cornexistin/Hydroxycornexistin.

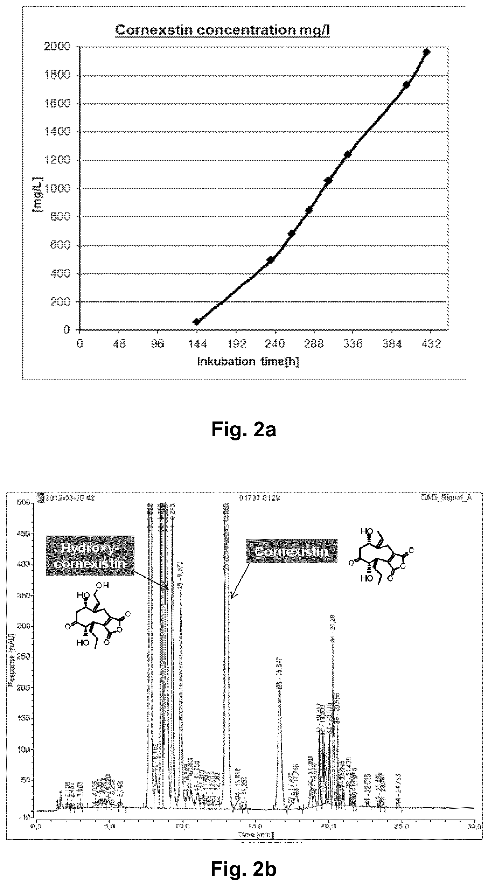

Figure 2a shows the accumulation of Cornexistin throughout the fermentation run. Figure 2b shows the HPLC chromatogram of the liquid phase of the fermentation broth illustrating the formation of Cornexistin and Hydroxycornexistin

### Removal of biomass

In order to remove biomass the fermentation broth containing Cornexistin/Hydroxycornexistin was centrifuged.

### Example 2.2: Fermentation of Paecilomyces divaricatus LU16891 and work-up for the production of Cornexistin and Hydroxycornexistin in 2.000 L volume

### a) Seed culture I

The strain Paecilomyces divaricatus LU16891 was grown in a two step fermentation. Cells were stored as spores after growth on potato agar. From the spore cell bank a first preculture was inoculated and grown in the following media designated S#5 (see above) :
8x 2L-Eerlenmeyer shaking flasks were filled with 400 mL S#5 Medium. They were each inoculated with 0,5ml of a frozen spore solution containing 3,1*10E8 spores/ml. The 2l Shake flasks without baffles covered with Kimguard cover were incubated for 96h at 26°C with an RPM of 220 and an amplitude of 4cm.

### b) Seed culture II

A 75L fermenter containing 36L of S#5 medium as described before wasp prepared. The medium was autoclaved for 60 min at 125°C. The pH was adjusted to pH6,7.

The seed culture II was performed as follows: The fermenter setting were done as followed: The aeration was set to 25 L/min, the pH value of the medium was set to 6,9 at the beginning. The dissolved pO₂ value was controlled at 30% by controlling the stirring speed. 3 disk plate stirrers were implemented in the fermenter, the lower stirrer speed was set to 100 rpm. During the fermentation the stirring speed was increased to keep the dissolve O₂ at 20%. pH was not controlled throughout fermentation. The 36 L Medium of the seed culture II were incubated for 33h as described in the 75I fermenter vessel. The fermenter was run at 0,5 bar overpressure and 26°C.

### c) Main culture

The composition of Main culture medium was the same as stated above.

### Preparation of main culture medium

Potato flakes, Glycerol and Pluriol P2000 were added to the fermenter and mixed with 1700 L demineralized water and autoclaved for 60min at 123°C. Urea solution was prepared as follows: 9200 g urea was dissolved in 40kg demineralized water and autoclaved at 121°C for 30min, the dissolved and sterile was added to the fermentation medium.

### Execution of main culture

The 36L volume of the seed culture II was inoculated in the 1700L of main culture medium.

The main culture was performed as followed: The incubation temperature was set to 26°C. The aeration was set to 25m³/h, the pH value of the medium was set to 5 by adding NaOH solution. The dissolved pO₂ value was controlled at 30% by controlling the stirring speed. 3 disk plate stirrers were implemented in the fermenter, the lower stirrer speed was set to 100 rpm. During the fermentation the stirring speed increased to a maximum of 200rpm. Pressure in the fermenter was to set to 0,5 bar. Glycerol concentration was kept above 10g/I throughout fermentation by adding amounts of glycerol continuously to the fermentation. Between 15d and 30d NH₃ gas was added to the fermenter to increase the pH to 5. After 31d the fermentation was terminated. Samples were withdrawn and prepared for the analysis of glycerol and Cornexistin/Hydroxycornexistin.

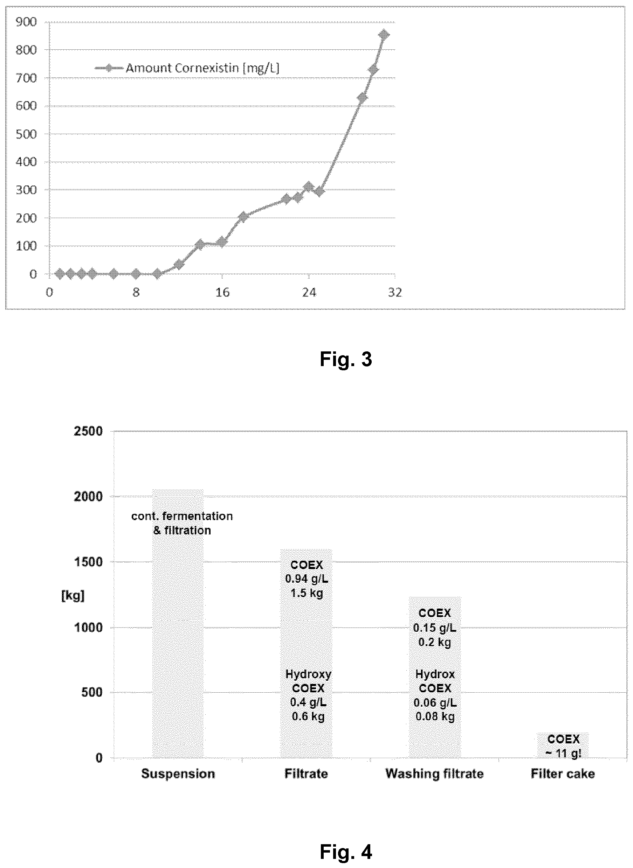

Figure 3 shows the accumulation of Cornexistin throughout the fermentation run in the 2000L scale.

### Example 2.3: Isolation of Cornexistin and Hydroxycornexisatin

### a) Removal of biomass

Due to the high filter resistances and consistency of the filter cake, a membrane filter press is recommended. Therefore, for the 2 m³ scale fermentation batch, a membrane filter press was used for removal of biomass.

Technical data of membrane filter press (filter plates from company Klinkau, filter press frame called "STRASSBURGER Wasch-Membranfilterpresse Type W-MFP 470/II E-C" from company Strassburger):
Number of chambers: 26 (13 membrane plates, 12 chamber plates, 1 head plate, 1 end plate)
Dimension of the plates: 470 x 470 mm
Chamber depth: 20 mm
Chamber volume: each ∼ 2,5 L
Filter area: per chamber ∼ 2.0,15 m²
Total filter area: ∼ 7,8 m²
Filter cloth: PP 2471, company Markert (calendered, air permeability 3 L/(dm^{2.}min) at 2 mbar)

The distribution of Cornexistin and Hydroxycornexistin as illustrated in Figure 4 was obtained.

Titers of ∼ 1g/L Cornexistin and 0.4 g/L Hydroxycornexistin were obtained in the filtrate. The amount of Cornexistin and Hydroxycornexistin in the washing filtrates is ∼ 11 wt% of the total amounts in both cases. Only 11 g = 0.7% of Cornexistin out of 1.66 kg are lost in the filter cake after filtration and one washing cycle.

7 filtration cycles for the membrane filtration were necessary (filtration with 6 bar pressure, pre-squeezing with 6 bar pressure, washing with 7.5 bar pressure, final squeezing with 7.5 bar, air blow down with 3-4 bar). 180 - 360 kg of fermentation broth were employed per cycle and a filtration time of 50 - 110 min, a pre-squeezing time of ∼ 20 min, a washing time of ∼ 15 - 20 min and a final squeezing and air blow down (in parallel) time of ∼ 60 min was observed.

### b) Adsorption chromatography of Cornexistin and Hydroxycornexistin

Starting material: The fermentation broth coming from the 2m³ run (see above) was was membrane filtered using a membrane filter press in order to remove biomass (see above). The thus obtained washing water and filtrate were used as starting materials for adsorption chromatograthy as described below.

250 L of Amberlite® XAD® 7 HP were filled into a chromatography vessel (Ø 500 x 1300 mm cyl. height) that was previously filled with 150 L dist. water. The resin was then washed with 5000 L dist. water at 1000 L/h. 1262 kg of washing water (Cornexistin content: 0.15 g/L = 0.2 kg, Hydroxycornexistin content: 0.06 g/L = 0.08 kg) resulting from the membrane filtration (brought to pH 2.5 with 10% sulfuric acid) were loaded with 400 L/h onto the column. Afterwards 1668 kg of the filtrate (Cornexistin content: 0.94 g/L = 1.5 kg, Hydroxycornexistin content: 0.4 g/L = 0.6 kg) (brought to pH 2.5 with 10% sulfuric acid) were loaded onto the column with 400 L/h. Then, 720 L dist. water were used to wash the column with 400 L/h. Elution was performed with ethyl acetate at 200 L/h.

Details of the adsorption chromatography and the observed yields of Cornexistin and Hydroxycornexistin are summarized in the table below. No losses are occurring during loading and washing. The only loss of ∼ 1% Cornexistin and ∼ 4% Hydroxycornexistin occurs in the aqueous phase of barrel 2 at the start of the elution process using ethyl acetate (see table below). Cornexistin was obtained via adsorption chromatography in a yield of 97 % and Hydroxycornexistin in a yield of 80% based on the mass and Cornexistin and Hydroxycornexistin contents in the eluate. The volume of the filtrate and washings after the membrane filtration of the fermentation broth was reduced by a factor of 4 using this adsorption chromatography technique.

| Amberlite XAD 7 | | | | | | | | |
|---|---|---|---|---|---|---|---|---|
| Sample | Mass | Content Cornexistin HPLC | pH | Cornexistin | Yield Cornexistin | Content HydroxyCornexistin HPLC | HydroxyCornexistin | Yield Hydroxycornexistin |
| | [kg] | [wt%] | | [kg] | [%] | [wt%] | [kg] | [%] |
| **Starting materials** | | | | | | | | |
| Loading wash water | 1262 | 0.015 | 2.2 | 0.189 | 11.2 | 0.006 | 0.076 | 10.7 |
| Loading filtrate | 1668 | 0.090 | 2.5 | 1.501 | 88.8 | 0.038 | 0.634 | 89.3 |
| **Loading of wash water** | | | | | | | | |
| 1 | 950 | 0 | 2.3 | 0 | 0 | 0 | 0 | 0 |
| 2 | 180 | 0 | 2.3 | 0 | 0 | 0 | 0 | 0 |
| 3 | 73 | 0 | 2.3 | 0 | 0 | 0 | 0 | 0 |
| **Loading of filtrate** | | | | | | | | |
| 1 | 979 | 0 | 2.7 | 0 | 0 | 0 | 0 | 0 |
| 2 | 186 | 0 | 2.7 | 0 | 0 | 0 | 0 | 0 |
| 3 | 180 | 0 | 2.7 | 0 | 0 | 0 | 0 | 0 |
| 4 | 195 | 0 | 2.7 | 0 | 0 | 0 | 0 | 0 |
| 5 | 97.5 | 0 | 2.7 | 0 | 0 | 0 | 0 | 0 |
| **Water wash** | | | | | | | | |
| 1 | 184 | 0 | 2.7 | 0 | 0 | 0 | 0 | 0 |
| 2 | 186 | 0 | 2.5 | 0 | 0 | 0 | 0 | 0 |
| 3 | 180 | 0 | 2.5 | 0 | 0 | 0 | 0 | 0 |
| 4 | 179.5 | 0 | 2.8 | 0 | 0 | 0 | 0 | 0 |
| **Elution with ethyl acetate** | | | | | | | | |
| Eluate Barrel 1 | 178.6 | 0 | 3.2 | 0 | 0 | 0 | 0 | 0 |
| Eluate Barrel 2 lower aqueous phase | 42.6 | 0.038 | 2.8 | 0.016 | 1.0 | 0.077 | 0.029 | 4.1 |
| Eluate Barrel 3 upper organic phase | 124 | 1.094 | 3.4 | 1.357 | 80.2 | 0.454 | 0.501 | 70.6 |
| Eluate Barrel 4 | 164.5 | 0.125 | 3.6 | 0.206 | 12.2 | 0.032 | 0.047 | 6.6 |
| Eluate Barrel 5 | 165.5 | 0.031 | 3.6 | 0.051 | 3.0 | 0.008 | 0.012 | 1.7 |
| Eluate Barrel 6 | 161.5 | 0.009 | 3.5 | 0.015 | 0.9 | 0.003 | 0.004 | 0.6 |
| Eluate Barrel 7 | 162.5 | 0.003 | 3.3 | 0.005 | 0.3 | 0 | 0 | 0 |
| Eluate Barrel 8 | 29.5 | 0.003 | 3.4 | 0.001 | 0.1 | 0 | 0 | 0 |
| | | | | | | | | |
| | | | | | | | | |
| Yield Cornexistin = | | 96.6 | | | | Yield Hydroxycornexistin = | | 79.5 |
| Loss Cornexistin = | | 1.0 | | | | Loss Hydroxycornexistin = | | 4.1 |
| Recovery Cornexistin = | | 97.6 | | | | Recovery Hydroxycornexistin = | | 83.6 |

Eluate barrel 3 - 6 (in total 616 kg) were taken further and the solvent ethyl acetate was removed in an 80 L reactor under a reduced pressure of 200 mbar at temperature (mantle) = 50 °C and temperature (in) = 35 °C with ∼ 20 L / h for ∼ 35 h. Finally 18.2 kg of a crude Cornexistin and Hydroxycornexistin solution in ethyl acetate was obtained containing 38 wt% of crude mass consisting of Cornexistin and Hydroxycornexistin. In detail, the dry mass was determined (by taking adequate fractions of the barrels) to be 6.85 kg of crude Cornexistin (which has a content of 24.1 wt% of dry mass = 1.65 kg) and Hydroxycornexistin (which has a content of 9.2 wt% of dry mass = ∼ 630 g).

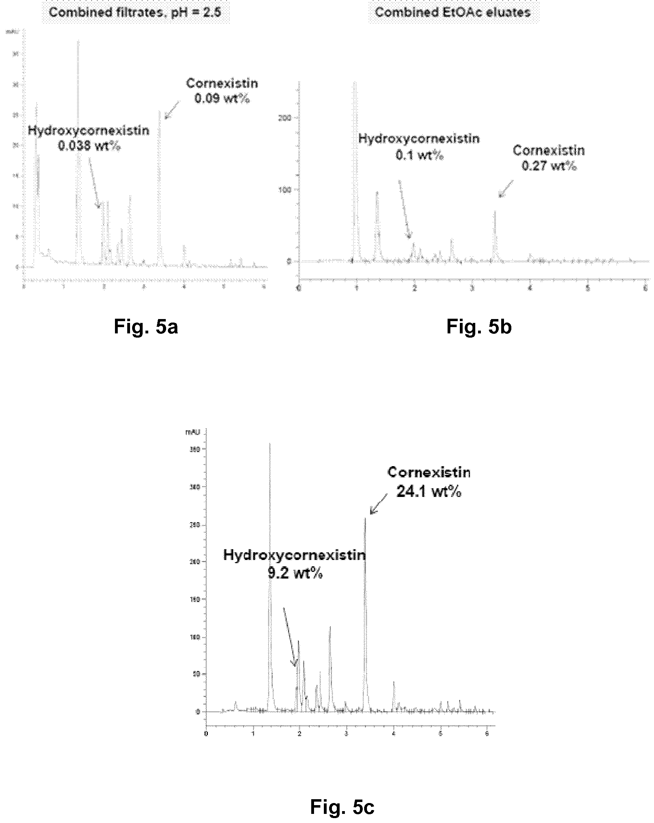

Figure 5a shows the HPLC analysis of the combined filtrates at pH 2.5 which are part of the starting material for the adsorption chromatography

Figure 5b shows the HPLC analysis of the combined eluates after adsorption chromatography.

### c) Silica gel chromatography

This material was further purified by silica gel chromatography according to the following conditions:
The ethyl acetate solution was concentrated under reduced pressure to yield 7.4 kg of crude material (corresponds to 40.9 wt% in ethyl acetate). Figure 5c shows the HPLC analysis of the combined eluates after adsorption chromatography and after removal of ethyl acetate.

Silica gel column purification was performed first using a gradient system of petroleum ether: acetone (10:1, 5:1, 4:1, 5:2, 2:1, 1:1). Thus, 1.8 kg of Cornexistin (40-50% purity) and 0.6 kg of Hydroxycornexistin (30-40% purity) was obtained in different fractions.

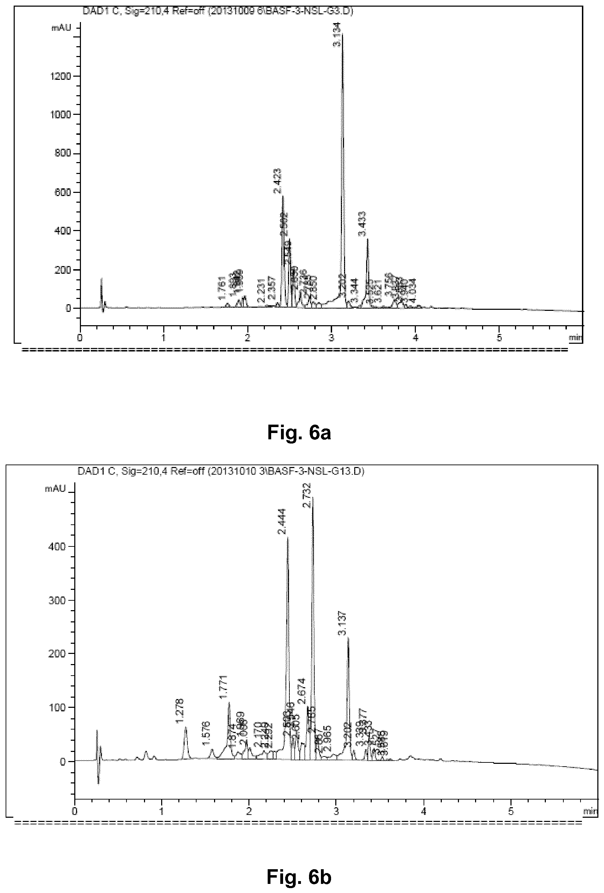

Figures 6a and b show the corresponding HPLC analysis of said two fractions containing Cornexistin (Fig. 6a)) and Hydroxycornexistin (Fig. 6b))

These fractions were further purified using HPLC purification according to the following details as described in the following sections.

### d) Preparative HPLC for Cornexistin:

| | |
|---|---|
| Column: | Luna C18 Gravity 5 µ m; length 500 mm, transversal section 50 mm; |
| Gradient: | Equilibrium: 2 Min. / 100mL/Min / 70 % water (+0.1% TFA) - 30% MeCN (+0.05% TFA) |
| Injection: | 100mL/Min / 80% water (+0.1% TFA) -20% MeCN(+0.1% TFA) - in 20.1 Min. > 50 % water (+0.1% TFA) -50% MeCN(+0.1 % TFA) |
| | |
| Pause: | 5 Min. / 100mL/Min / 50 % water (+0.1% TFA) - 50% MeCN(+0.1 % TFA) |
| Wash column: | 2 Min. / 100mL/Min- 100% MeCN(+0.1 % TFA) |

### e) Preparative HPLC for Hydroxycornexistin:

| | |
|---|---|
| Column: | Luna C18 Gravity 5 µ m; length 500 mm, transversal section 50 mm; |
| Gradient: | Equilibrium: 2 Min. / 100mL/Min/85 % water (+0.1% TFA) - 15% MeCN (+0.1% TFA) |
| Injection: | 100mL/Min / 85% water (+0.1% TFA) -15% MeCN -in 30.1 Min. > 55 % water (+0.1% TFA) - 45% MeCN(+0.1 % TFA) |
| Pause: | 5 Min. / 100mL/Min / 55 % water (+0.1% TFA) -45% MeCN(+0.1 % TFA) |
| Wash column: | 2 Min. / 100mL/Min - 100% MeCN(+0.1% TFA) |

Via preparative HPLC 950 g of Cornexistin with a purity of 95 HPLC-a% and 220 g of Hydroxycornexistin with a purity of 87 HPLC-a% were obtained which corresponds to a yield of 58% for Cornexistin and 35% for Hydroxycornexistin for normal and reverse phase chromatography purification.

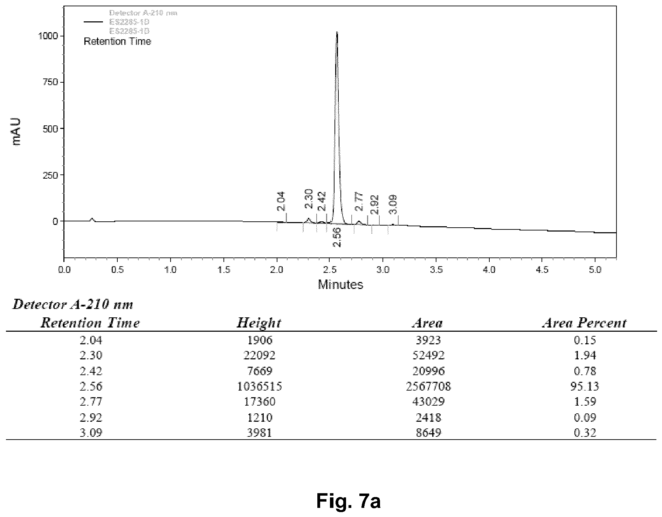

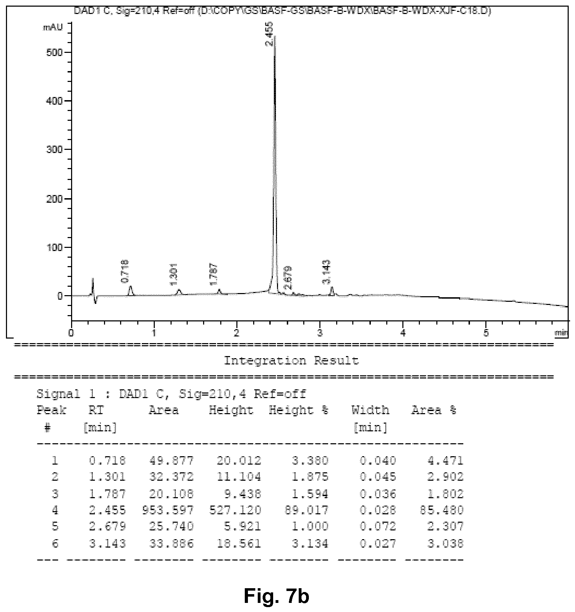

Figures 7a and b show the corresponding HPLC analysis of the obtained Cornexistin (Fig. 7a)) and Hydroxycornexistin (Fig. 7b))

The entire content of the documents as cited herein is incorporated by reference.

## Claims

1. A process for isolating an aliphatic, mono- or polycyclic small molecule from the aqueous fermentation broth of an eukaryotic microorganism, comprising
a) performing a solid/liquid separation step in order to remove biomass from the fermentation broth;
b) contacting the obtained liquid phase of step a) with a first porous adsorbent, which adsorbs said small molecule,
c) desorbing the small molecule by eluting the adsorbent with an organic solvent, and
d) isolating the active ingredient from the eluate.

2. The process according to claim 1, wherein said small molecule is a biocide.

3. The process of claim 1 or 2, wherein the first porous adsorbent as used in step b) is an aliphatic or aromatic polymeric adsorbent, a basic adsorbent, an acidic adsorbent or charcoal.

4. The process of claim 3, wherein the first porous adsorbent is a polyacrylate-based or a polyaromatic-based, in particular polystyrene-based, adsorbent or a polyacrylate- and polyaromatric-based adsorbent.

5. The process of claim 4, wherein said adsorbent is **characterized by** at least one of the following parameters:
b) pore diameter in the range of 55 to 550 Angstrom;
c) surface area in the range of 300 to 800 m²/g;
d) porosity of at least 0,50 ml/ml;
e) particle size in the range of 0,2 to 2,5 mm;

6. The process of claim 4 or 5, wherein a) said aliphatic or aromatic polymeric adsorbent adsorbent is Amberlite ®XAD® 7 HP, Amberlite ®XAD®1180 or Treversorb ADS700; b) said basic adsorbent is selected from Ambersep 900 OH, Amberlite IRA 900 CI or Lewatit MP 62; c) said acidic adsorbent is selected from Amberlite ®IRC 86, Amberlite 252H or Lewatit ® K2621; or d) said charcoal is selected from Norit C Gran and CAL ® Chemviron.

7. The process of one of the preceding claims, wherein the fermentation broth or the liquid phase of step a) is subjected to a pH adjustment.

8. The process of one of the preceding claims, wherein the eluate of step c) is contacted with a second adsorbent which adsorbs the small molecule.

9. The process of claim 8, wherein said second adsorbent is silica gel.

10. The process of claim 8 or 9, wherein the small molecule is desorbed by eluting the second adsorbent with an organic solvent.

11. The process of one of the preceding claims, wherein said small molecule is selected from Cycloclavine and Cornexistin/Hydroxycornexistin.

12. The process of one of claims 1 to 11 for isolating Cycloclavine from the aqueous fermentation broth of an eukaryotic microorganism, comprising
a) performing a solid/liquid separation step in order to remove biomass from the fermentation broth;
b) contacting the obtained liquid phase of step a) with a first porous adsorbent, which adsorbs Cycloclavine,
c) desorbing the Cycloclavine by eluting the adsorbent with an organic solvent, and
d) isolating the active ingredient from the eluate.

13. The process claim 12, wherein the eukaryotic microorganism is a microorganism of the genus *Saccharomyces,* in particular *S. cerevisiae.*

14. The process of anyone of claims 12 or 13, wherein the fermentation broth or the liquid phase of step a) is alkalized, preferably adjusted to a pH in the range of 8 to 11, preferably 9 to 10.

15. The process of anyone of the claims 12 to 14, wherein the first porous adsorbent as used in step b) is an aliphatic polymeric adsorbent, in particular a polyacrylate-based adsorbent and preferably Amberlite ®XAD® 7 HP.

16. The process of anyone of the claims 12 to 15, wherein Cycloclavine is desorbed from the adsorbent by washing with a polar organic solvent, in particular, ethylacetate, MTBE, THF, acetone or mixtures of at least two of them.

17. The process of anyone of the claims 12 to 16, wherein the eluate from the first adsorbent or a crude product obtained from said eluate by removal of the solvent and redissolving in a more non-polar second solvent, in particular mixtures of MTBE and cyclohexane, preferably in a ratio (vol/vol) of 1 : 1, 2 : 1, or 1 : 2, is contacted with a second adsorbent, in particular silica gel, which adsorbs Cycloclavine .

18. The process of claim 17, wherein Cycloclavine is desorbed by washing the second adsorbent with an organic solvent, in particular MTBE.

19. The process of one of the claims 1 to 11 for isolating Cornexistin and/or Hydroxycornexistin from the aqueous fermentation broth of an eukaryotic microorganism, comprising
a) performing a solid/liquid separation step in order to remove biomass from the fermentation broth;
b) contacting the obtained liquid phase of step a) with a first porous adsorbent, which adsorbs Cornexistin and/or Hydroxycornexistin,
c) desorbing the Cornexistin and/or Hydroxycornexistin by eluting the adsorbent with an organic solvent, and
d) isolating the active ingredient from the eluate.

20. The process of claim 19, wherein the eukaryotic microorganism is a microorganism of the genus *Paecilomyces,* in particular *P. divaricatus.*

21. The process of anyone of claims 19 or 20, wherein the fermentation broth or the liquid phase of step a) is acidified, preferably adjusted to a pH in the range of 1 to 4, preferably 2 to 2.5.

22. The process of anyone of the claims 19 to 21, wherein the first porous adsorbent as used in step b) is an aliphatic polymeric adsorbent, in particular a polyacrylate-based adsorbent and preferably Amberlite ®XAD® 7HP or Treversorb ADS700; or is an aromatic polymeric resin, preferably a polystyrene-based resin, like Amberlite ®XAD®1180

23. The process of anyone of the claims 19 to 22, wherein Cornexistin and/or Hydroxycornexistin is desorbed from the adsorbent by washing with a polar organic solvent, in particular, ethyl acetate, MTBE, THF, acetone or mixtures thereof.

24. The process of anyone of the claims 19 to 23, wherein the eluate from the first adsorbent or a crude product obtained from said eluate by removal of the solvent redissolving in a more non-polar second organic solvent is contacted with a second adsorbent, in particular silica gel, which adsorbs Cornexistin and/or Hydroxycornexistin.

25. The process of claim 23 or 24, wherein Cornexistin and Hydroxycornexistin are desorbed separately by washing the second adsorbent with an organic solvent, in particular by applying a solvent gradient of petroleum ether: acetone, in particular in a range of about 10:1, to 1:1, preferably comprising the steps 10:1, 5:1, 4:1, 5:2, 2:1, and 1:1.

26. The process of claim 25, wherein Cornexistin and/or Hydroxycornexistin are further purified by RP PHLC, in particular by applying a C18 reversed phase column with acetonitrile-water-trifluoroacetic acid or acetonitrile-water-formic acid as eluent.

27. The process of claim 26, wherein Cornexistin is purified by applying the following gradient: 70 % water (+0,1% TFA) / 30% MeCN (+0,05% TFA), 80 % water (+0,1% TFA) / 20% MeCN (+0,1% TFA), and 50 % water (+0,1% TFA) / 50% MeCN (+0,1% TFA);

28. The process of claim 26, wherein Hydroxycornexistin is purified by applying the following gradient: 90 % water (+0,1% TFA) / 10% MeCN (+0,05% TFA), 90 % water (+0,1% TFA) / 10% MeCN (+0,1% TFA), 60 % water (+0,1% TFA) / 40% MeCN (+0,1% TFA)).

29. A small molecule, in particular Cycloclavin, Cornexistin and/or Hydroxycornexistin or a product containing at least one of small molecules, obtainable by a process of anyone of the preceding claims.

30. A biocidal composition comprising at least one compound of claim 29, optionally in combination with a further biocidally active constituent.
